# EUROPEAN PATENT APPLICATION

(11) **EP 2 368 506 A1**
(43) Date of publication of application: **28.09.2011**
(21) Application number: 11004993.9
(22) Date of filing: 18.09.2009
(51) Int. Cl.: A61B 17/70, A61B 17/80

(54) **Anterior transpedicular screw-and-plate system**

(30) Priority: 18.09.2008 US 9803 P
(62) Divisional of application: 09792704.0
(71) Applicant: Synthes GmbH, 4436 Oberdorf (CH)
(72) Inventor: Overes, Thomas, 4513 Langendorf (CH); Zurschmiede, Silas, 2540 Grenchen (CH); Frigg, Robert, 2544 Bettlach (CH); Lechmann, Beat, 2540 Grenchen (CH)
(74) Representative: Lusuardi, Werther

(57) **Abstract**

An anterior transpedicular bone fixation device (100) includes a first plate (105) having at least one fastener hole (120a) configured to receive at least a portion of a first bone fastener and a first rotatable eccentric member (112). The first eccentric member has a first aperture (106) for receiving at least a portion of a central fastener (108). A second plate (110) has at least one fastener hole (120c) configured to receive at least a portion of a second bone fastener and a second rotatable eccentric member (114) with a second aperture (107) for receiving at least a portion of the central fastener, wherein the first and second rotatable eccentric members enable the first plate and second plate to translation with respect to one another. The orientation of the first plate with respect to the second plate can be fixed by advancing the central fastener through the apertures.

## Description

### CROSS-REFERENCE TO RELATED APPLICATIONS

This application claims the benefit of U.S. Provisional Application No. 61/098,036, filed on September 18, 2008, entitled "ANTERIOR TRANSPEDICULAR SCREW-AND-PLATE SYSTEM," the contents of which is incorporated in its entirety by reference herein.

### BACKGROUND OF THE INVENTION

Multilevel cervical spinal procedures result in relatively large loads on anterior cervical screw and plate systems, particularly in cases of severe three-column subaxial cervical spinal injuries and multilevel plated reconstructions in osteoporotic bone. Supplemental posterior instrumentation is therefore recommended to increase primary construct rigidity and limit potential compromise of the screw and plate systems. The increasing number of successfully performed posterior cervical pedicle screw fixations have enabled more stable fixations, however, most cervical pathologies are located anteriorly and are preferably addressed by an anterior approach.

Additionally, the use of pedicle screw fixations in the vertebrae area are typically limited by the position of the screw because improperly placing the screw results in potential arterial or spinal cord damage.

Thus there is a need for a bone plating system that combines the advantages of an anterior approach with the superior biomechanical characteristics of a cervical pedicle screw fixation and expands the translation of the bone plating system while maintaining the proper bone screw placement.

### SUMMARY OF THE INVENTION

The present invention relates generally to a bone plate. More specifically, the present invention relates to a cervical anterior transpedicular bone fixation device. A bone fixation device according to one embodiment of the present invention comprises a first plate having an upper surface and a lower surface, at least one fastener hole configured to receive at least a portion of a first bone fastener, the fastener hole extending from the upper surface through to the lower surface, and a first rotatable eccentric member, the first eccentric member having a first aperture for receiving at least a portion of a central fastener, a second plate having an upper surface and a lower surface, at least one fastener hole configured to receive at least a portion of a second bone fastener, the fastener hole extending from the upper surface through to the lower surface, and a second rotatable eccentric member, the second eccentric member having a second aperture for receiving at least a portion of the central fastener, wherein the first and second rotatable eccentric members enable the first plate and second plate to vertically translate with respect to one another, and the orientation of the first plate with respect to the second plate can be fixed by advancing the central fastener through the first and second apertures. In one preferred embodiment, the first and second rotatable eccentric members further enable the first plate and second plate to experience horizontal translation with respect to one another.

According to one embodiment, the bone fixation assembly attains its maximum length along the central longitudinal axis of the bone assembly when the first aperture is in its furthest position along the central longitudinal axis from the at least one fastener hole of the upper plate and the second aperture is in its furthest position along the central longitudinal axis from the at least one fastener hole of the lower plate and attains its minimum length along the central longitudinal axis when the first aperture is in its closest position along the central longitudinal axis to the at least one fastener hole of the upper plate and the second aperture is in its closest position along the central longitudinal axis to the at least one fastener hole of the lower plate.

In another embodiment, a bone fixation system is disclosed where the bone fixation system comprises a plate comprising a top surface, a bottom surface and at least one fixation hole extending between the top and bottom surfaces, the bone fixation system further comprising at least one rotatable eccentric compression ring disposed in at least a portion of the at least one fixation hole and at least one fastener comprising a head and a threaded shaft; wherein the at least one rotatable eccentric compression ring is configured and dimensioned to permit the plate to translate relative to the at least one fastener.

In one preferred embodiment, the plate has a plurality of fixation holes and at least one of said plurality of fixation holes has a concentric compression ring disposed therein.

In one preferred embodiment, at least one fastener of the bone fixation system comprises: a threaded shaft and a head, the head including a radial wall and open end defining a recess for insertion of a threaded locking screw wherein when the threaded locking screw is threaded into the head of the fastener, the radial wall is expanded outward to interact with a wall of the eccentric compression ring.

### BRIEF DESCRIPTION OF SEVERAL VIEWS OF THE DRAWINGS

The foregoing summary, as well as the following detailed description of preferred embodiments of the application, will be better understood when read in conjunction with the appended drawings. For the purposes of illustrating the device of the present application, there is shown in the drawings preferred embodiments. It should be understood, however, that the application is not limited to the precise arrangement, structures, features, embodiments, aspects, and instrumentalities shown, and the arrangements, structures, features, embodiments, aspects and instrumentalities shown may be used singularly or in combination with other arrangements, structures, features, embodiments, aspects and instrumentalities. In the drawings:

Fig. 1 illustrates a top perspective view of an assembled plate according to a first preferred embodiment of the present invention;

Fig. 2 illustrates a bottom perspective view of the assembled plate of Fig. 1;

Figs. 3A illustrates a cross-sectional view of the assembled plate of Fig. 1, taken generally perpendicular to a longitudinal axis of the plate and through a central screw;

Fig. 3B illustrates a magnified cross-sectional view taken adjacent the central screw of Fig. 3A;

Fig. 4A illustrates a top perspective view of the assembled plate of Fig. 1 with upper and lower plates slightly reshaped and resized;

Fig. 4B illustrates a top perspective view of the assembled plate of Fig. 4A in a partially expanded position;

Fig. 4C illustrates a top perspective view of the assembled plate of Fig. 4A in a fully expanded position;

Fig. 5A illustrates a top plan, partially exploded view of the assembled plate of Fig. 4A with upper and lower plates slightly reshaped and resized;

Fig. 5B illustrates a top plan, partially exploded view of the assembled plate of Fig. 4B with upper and lower plates slightly reshaped and resized;

Fig. 5C illustrates a top plan, partially exploded view of the assembled plate of Fig. 4C with upper and lower plates slightly reshaped and resized;

Fig. 6 illustrates a top perspective view of the plate of Fig. 1 mounted to an anterior portion of a spine;

Fig. 7A illustrates a top perspective view of an assembled plate according to a second preferred embodiment of the present invention;

Fig. 7B illustrates a top perspective, magnified, fragmentary view of an assembled plate of Fig. 7A;

Figs. 8A-8G illustrates method steps of mounting bone fasteners to the assembled plate of Fig. 7A;

Fig. 9 illustrates a top perspective view of the plate of Fig. 7A mounted to an anterior portion of the spine.

Fig. 10A illustrates a top perspective view of a partially assembled plate and bone fasteners according to a third preferred embodiment of the present invention;

Fig. 10B illustrates a top perspective view of the assembled plate and bone fasteners of Fig. 10A; and

Fig. 10C illustrates a top perspective view of the assembled plate and bone fasteners of Fig. 10A.

### DETAILED DESCRIPTION OF CERTAIN EMBODIMENTS

Certain terminology is used in the following description for convenience only and is not limiting. The words "right", "left", "top" and "bottom" designate directions in the drawings to which reference is made. The words "inwardly" and "outwardly" refer to directions toward and away from, respectively, the geometric center of the facet interference screw and designated parts thereof. The words, "anterior", "posterior", "superior", "inferior", "lateral", "sagittal", "axial", "coronal" and related words and/or phrases designate preferred positions and orientations in the human body to which reference is made and are not meant to be limiting. The terminology includes the above-listed words, derivatives thereof and words of similar import.

Certain embodiments of the present invention will now be discussed with reference to the aforementioned figures, wherein like reference numerals refer to like components. Preferred embodiments of the present invention are directed to a cervical anterior transpedicular screw-and-plate system. However, the preferred embodiments of the screw-and-plate system are not limited to applications or mounting in the anterior spine and may be utilized in the lumbar spine or for mounting to other bones in the human body, as would be apparent to one having ordinary skill in the art.

The plates described herein may be used in spinal fusion procedures in which a damaged or diseased disc (or part of a disc) is removed from between a pair of vertebrae and a spinal fusion spacer is placed between the vertebrae. The plates are applied to an anterior portion of the affected vertebrae to span the affected disc space, and may be fixed to the vertebrae using bone screws as will be described in more detail below. The plate functions to maintain the vertebrae aligned during the initial period following fixation in which fusion of the spacer to the adjacent vertebrae occurs. The plate may also function to share some of the axial spinal load applied to the fusion spacer to prevent extreme subsidence of the spacer into the vertebral body, such as where the patient has poor bone quality. The plates may also act to prevent the spacer from being expelled from the disc space during the initial post-operative period.

The plates may be used for single level (i.e. one-disc) fusion procedures, although in second and third preferred embodiments, the plates are used in multiple-level (i.e. multiple discs) fusion procedures. Some embodiments may be used for corpectomy procedures, in which at least a portion of a vertebral body is removed. While the plates herein are described with reference and application to the spine, it will be appreciated that features of the plates and the plates may have other applications, and can be applied to other bones and/or parts of the skeleton.

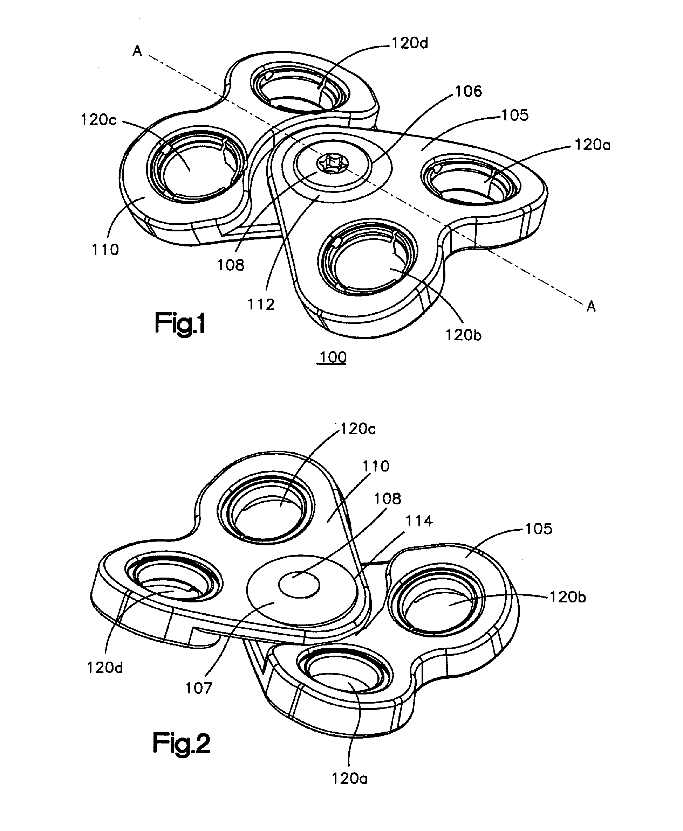

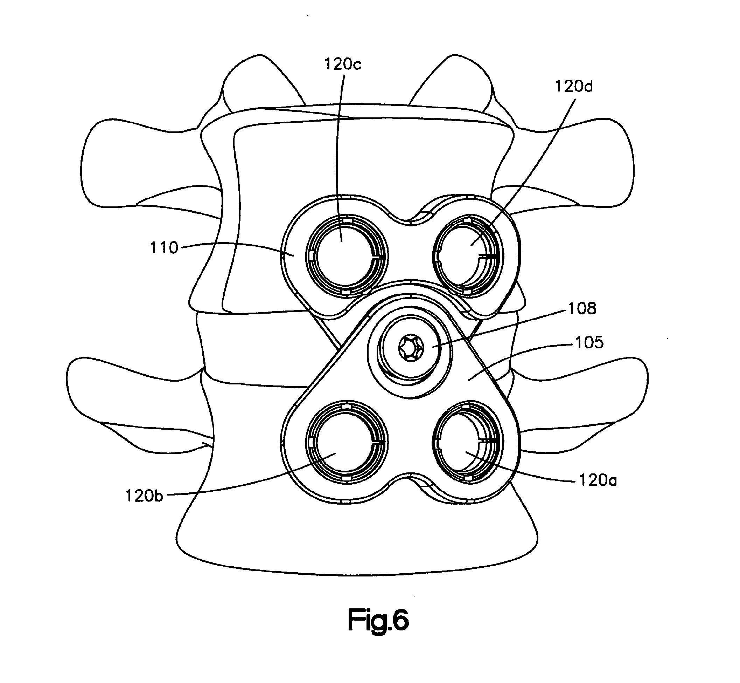

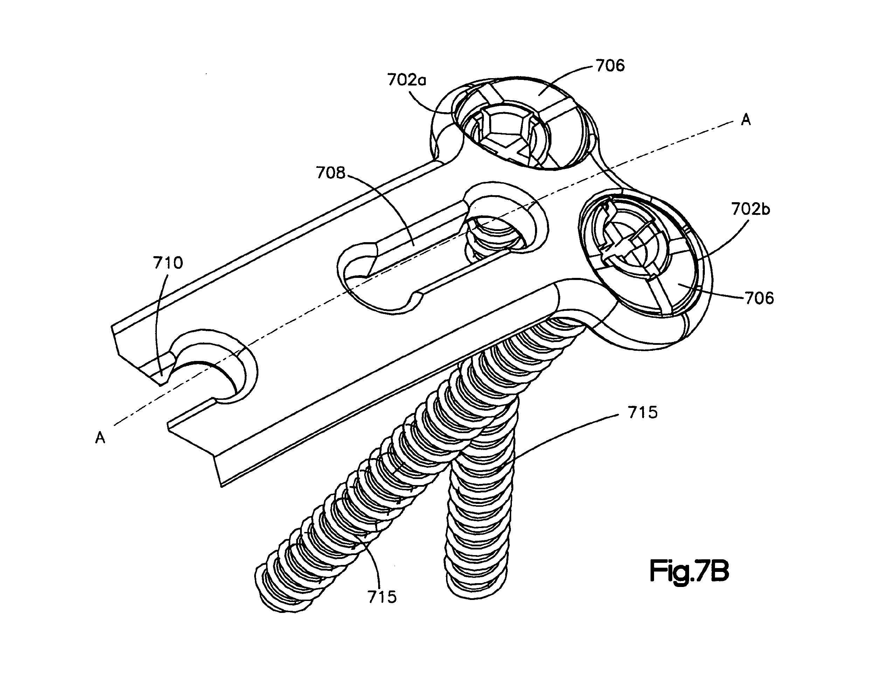

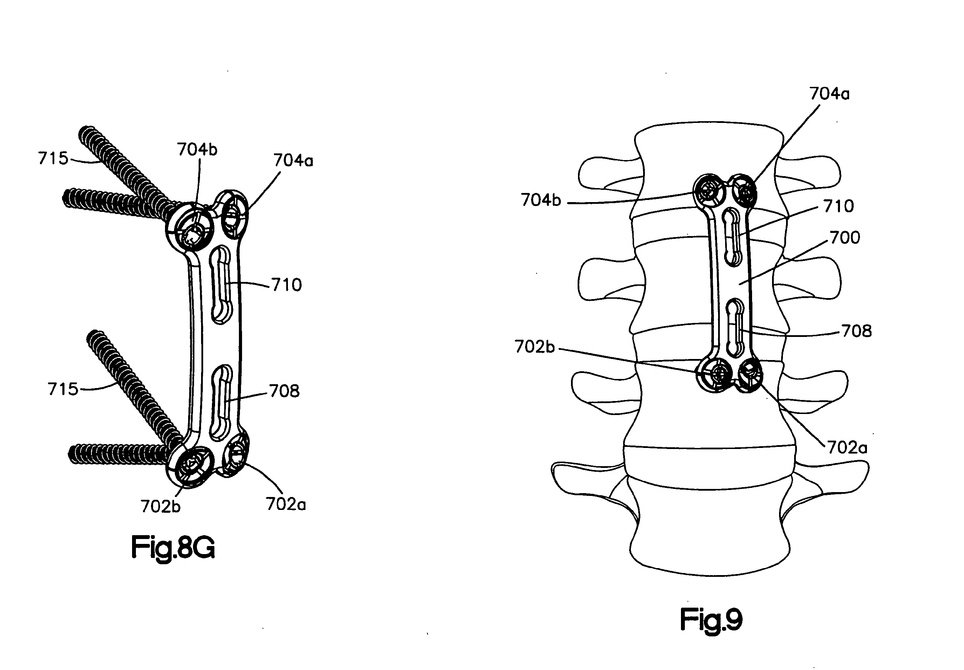

Referring to Figs. 1-6, a first embodiment of the system includes a locking plate 100, which has an upper plate 105, a lower plate 110, and a longitudinal axis A-A. The upper plate 105 has a rotatable, ring-shaped eccentric member 112, which contains an off-center aperture 106, and the lower plate 110 has a rotatable ring-shaped eccentric member 114 that contains an off-center aperture 107 for rotatably interconnecting the upper plate 105 to the lower plate 110 via a central screw 108. The first preferred embodiment of locking plate 100 includes two fastener holes 120a, 120b at an end of upper plate 105 and two fastener holes 120c and 120d at an end of lower plate 110. The fastener holes 120a, 120b, 120c, 120d may be configured to receive at least a portion of a bone fastener (See, for example, bone fastener 715 (Fig. 7B)), which may be inserted into a bone segment, such as a vertebral body (See Figs. 6 and 9). Although the plate 100 is shown with two pairs of fixation holes 120a, 120b, 120c, 120d, more than two pairs may be provided, for example so that plate 100 may span a greater length and thus be fastened to multiple locations along the spine or across multiple levels. Single holes (not shown), alternatively, may be provided as opposed to the pairs of fastener holes 120a, 120b, 120c, 120d. Additionally, each fastener hole 120a, 120b, 120c, 120d may contain a compression ring (not shown), as would be apparent to one having ordinary skill in the art, to receive the bone fasteners and the plate 100 may also have one or more visualization windows (not shown) extending from the upper surface of upper plate 105 through the lower surface of upper plate 105. The window may provide visual access to a disc space below the plate 100 when implanted into a patient's body.

Fig. 2 shows the under surface of the upper and lower plates 105, 110 in more detail. As can be seen in Fig. 2, the upper plate 105 is rotatably interconnected with the lower plate 110 via the insertion of the central screw 108 through both of the off-center apertures 106, 107 of the upper and lower plates 105, 110. Once the screw 108 is fully inserted through both of the off-center apertures 106, 107, the upper and lower plates 105 and 110 are secured to each other.

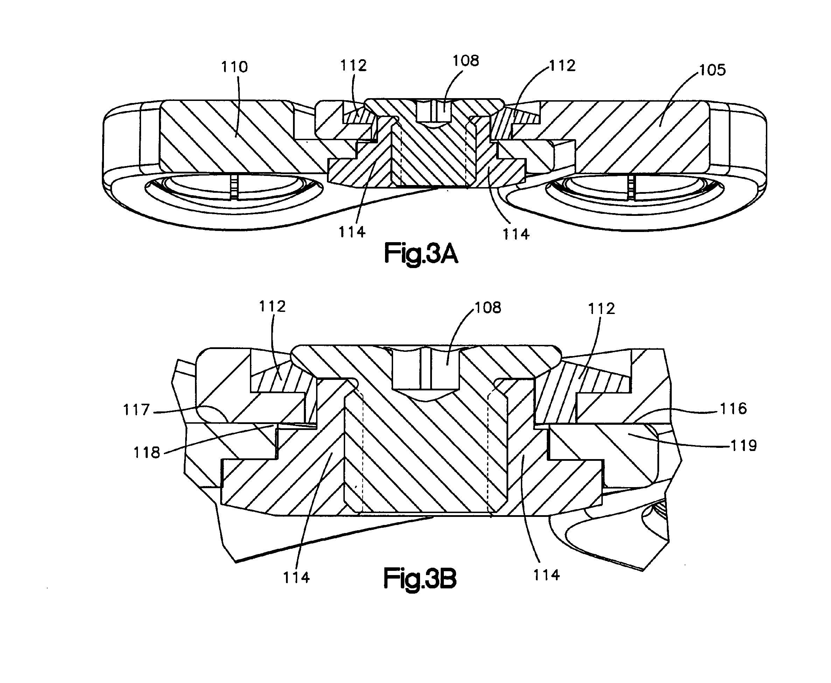

As can be seen in Figs. 3A and 3B, as the screw 108 is inserted through the off-center apertures 106, 107 and the screw 108 is tightened, the upper and lower plates 105, 110 are drawn closer together locking the position of the upper and lower plates 105, 110 relative to each other. Preferably, contact areas or surfaces 116, 117 of the upper plate 105 and contact areas or surfaces 118, 119 of lower plate 110 that contact each other in an assembled configuration may include a certain roughness, which allows the contact surfaces 116, 117, 118, 119 to exert friction relative to each other so that when the screw 108 is inserted though through the off-center apertures 106, 107 in the ring-shaped eccentric members 112, 114, the upper plate 105 and the lower plate 110 are secured to each other, thereby controlling the transverse sliding of the lower plate 105 with respect to the upper plate 110 along the longitudinal axis A-A of the plate 100.

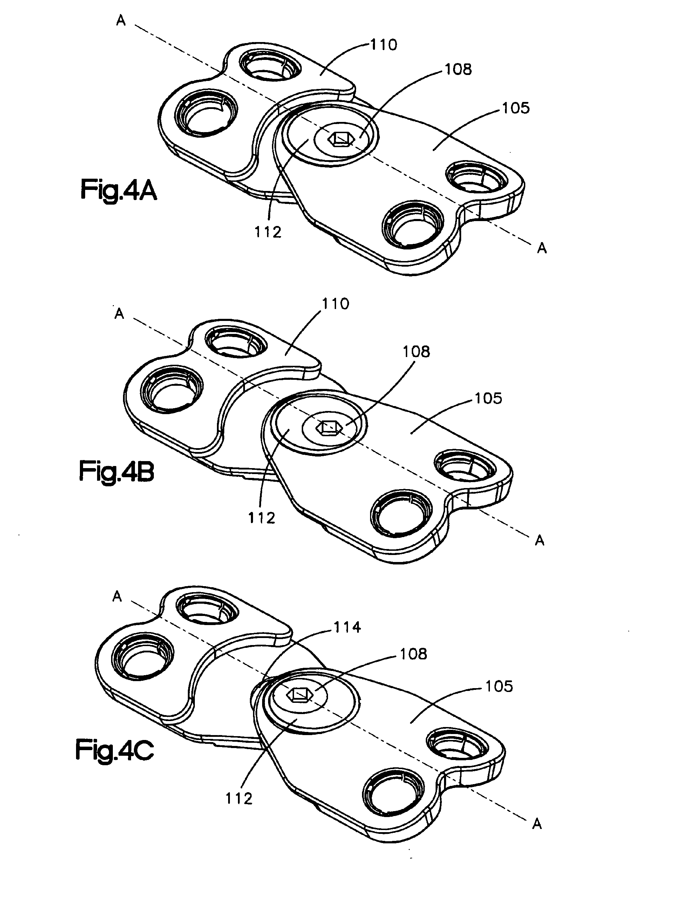

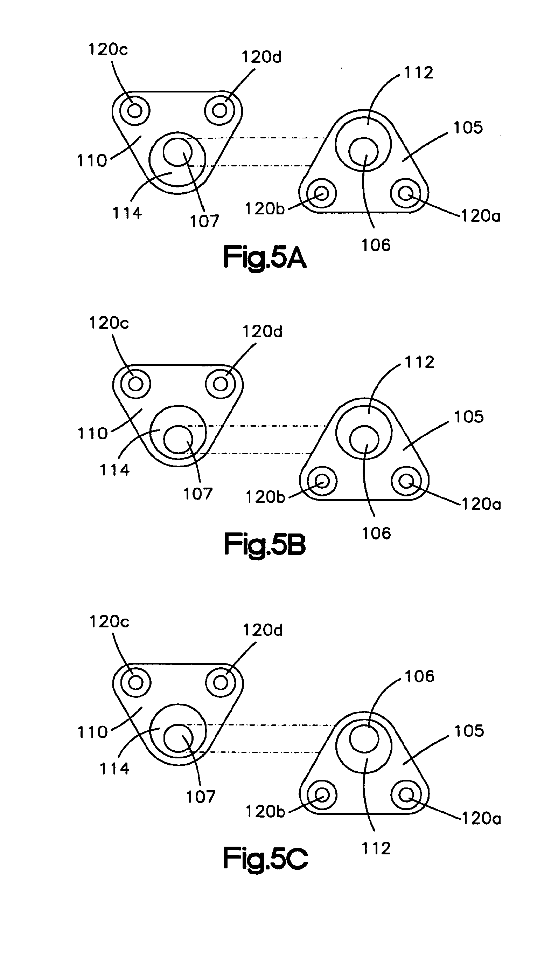

Each of the eccentric members 112, 114 are preferably rotatable relative to their respective upper and lower plates 105, 110. As can be seen in Figs. 4A and 5A, when the eccentric members 112, 114 are in their original positions or when the off-center aperture 106 of the upper plate 105 is in its closest position to a distal end of the upper plate 105 and the off-center aperture 107 of the lower plate 110 is in its closest position to a distal end of the lower plate 110, the screw plate 100 has its minimum length along the longitudinal axis A-A. In order to expand the length of the plate 100, the off-center eccentric members 112, 114 can be rotated individually or in concert to allow the upper and lower plates 105, 110 to translate relative to each other.

As can be seen in Figs. 4B and 5B, when the off-center eccentric member 114 of the lower plate 110 is rotated, thereby moving the off-center aperture 107 further away from the distal end of the lower plate 110, and closer to the distal end of the upper plate 105, the lower plate 110 is translated away from the upper plate 105 creating a greater length along the longitudinal axis A-A of the plate 100. Similarly, when the off-center eccentric member 112 of the upper plate 105 is rotated, thereby moving the off-center aperture 106 further away from the distal end of the upper plate 105 and closer to the distal end of the lower plate 110, the upper plate 105 is translated away from the lower plate 105 creating a greater length along the longitudinal axis A-A of the plate 100. Thus, as the off-center eccentric members 112, 114 are rotated in varying degrees, the length of the plate 100 along the longitudinal axis A-A is varied. As can be seen in Figs. 4C and 5C, when the eccentric member 114 of the lower plate 110 is rotated to allow the aperture 107 of the lower plate 110 to be in its furthest position along the longitudinal axis A-A relative to the distal end of the lower plate 110 and the off-center eccentric member 112 of the upper plate 105 is rotated to allow the aperture 106 of the upper plate 105 to be in its furthest position along the longitudinal axis A-A relative to the distal end of the upper plate 105, the plate 100 attains its greatest length along the longitudinal axis A-A.

Similarly, as will be appreciated by one of ordinary skill in the art, the upper and lower plates 105, 110 can attain varying horizontal translation relative to each other and the longitudinal axis A-A by rotating either or both of the eccentric members 112, 114 so that the apertures 106, 107 are positioned at various locations along a horizontal axis of plate 100, wherein the horizontal axis is generally perpendicular to the longitudinal axis A-A.

In conventional plating systems, a surgeon typically needs to rely on several different sized bone plates in order to account for the large number of possible dimensions of a patient's anatomy. In contrast, the use of the eccentric members 112, 114 of the plate 100 of the first preferred embodiment enables the use a limited number of different bone plates to account for the differing dimensions of a patient's anatomy. For instance, the below calculation illustrates how it is possible, through the rotation of the eccentric members 112, 114, to encompass a vast array of vertical distances with a limited number of plates. Where the eccentricity of the eccentric member 112 (e.g., in mm) of the upper plate 105 equals a distance between a center of the eccentric member 112 of the upper plate 105 and a center of the aperture 106 of the upper plate 105 and the eccentricity of the eccentric member 114 (e.g., in mm) of the lower plate 110 equals a distance between the center of eccentric member 112 of the lower plate 110 and a center of the aperture 107 of the lower plate 110, the maximal variability of the plate 100 along the longitudinal axis A-A would equal two times the eccentricity of the eccentric member 112 of the upper plate 105 plus two (2) times the eccentricity of the eccentric member 114 of the lower plate 110 ((2*eccentricity 112) + (2*eccentricity 114)). As mentioned above, the length of the plate 100 along the longitudinal axis A-A can be varied by rotating each or both of the eccentric members 112, 114 resulting in a single plate 100 that can be used for patients with varying anatomical dimensions.

One exemplary surgical technique for implanting the plate 100 is described below, however, those skilled in the art will appreciate that the plate 100 utilizing numerous techniques and/or surgical steps that would be apparent to one having ordinary skill in the art, following a review of the present disclosure.

In use, for implantation of the plate 100 in the middle and lower cervical spine, an antero-lateral approach is preferred. If the plate 100 is to be extended over several segments of the spine, a long incision is preferred. When exposing the vertebral bodies, the anterior longitudinal ligament is preferably removed or incised only in the areas where the intervertebral disc is to be bridged by the plate 100. Such a technique limits damage to the anterior longitudinal ligament in adjacent segments.

After the incision has been made, an image intensifier tool, such as Fluoroscopy (not shown), may be used to guide and monitor guide wires which are placed in the vertebra, preferably from the anterior side. Using spreaders, the cranial and caudal end-segments of the spine are spread and a corpectomy implant or natural bone is implanted to replace the removed segments. The distance between the guide wires is measured with, for example, a caliper instrument (not shown), which measurement can be utilized to determine plate size. Once the distance is determined, the plate 100 is preferably adjusted to the ideal length by rotating the eccentric members 112, 114 of the upper and lower plates 105, 110 and the adjusted plate 100 is placed over the guide wires. Once the proper size has been determined, before or after screws or fasteners have been inserted into the patient, the plate 100 is preferably locked by tightening the central screw 108.

To affix the plate 100 to the patient's vertebrae, cannulated screws are preferably guided over the guide wires and inserted through the fastener holes 120a, 120b, 120c, 120d. Although cannulated screws are preferably used, any heretofore known or hereafter developed means of fixation can be used. For example, cortical, pedicle or spongiosa screws may be placed into the vertebral body to fasten the plate 100 thereto. Additionally, once the fasteners or screws have been inserted, the screws can be locked using locking screws. An exemplary use of locking screws is disclosed in U.S. Patent No. 6,235,033, entitled "Bone Fixation Assembly", the contents of which are incorporated herein by reference.

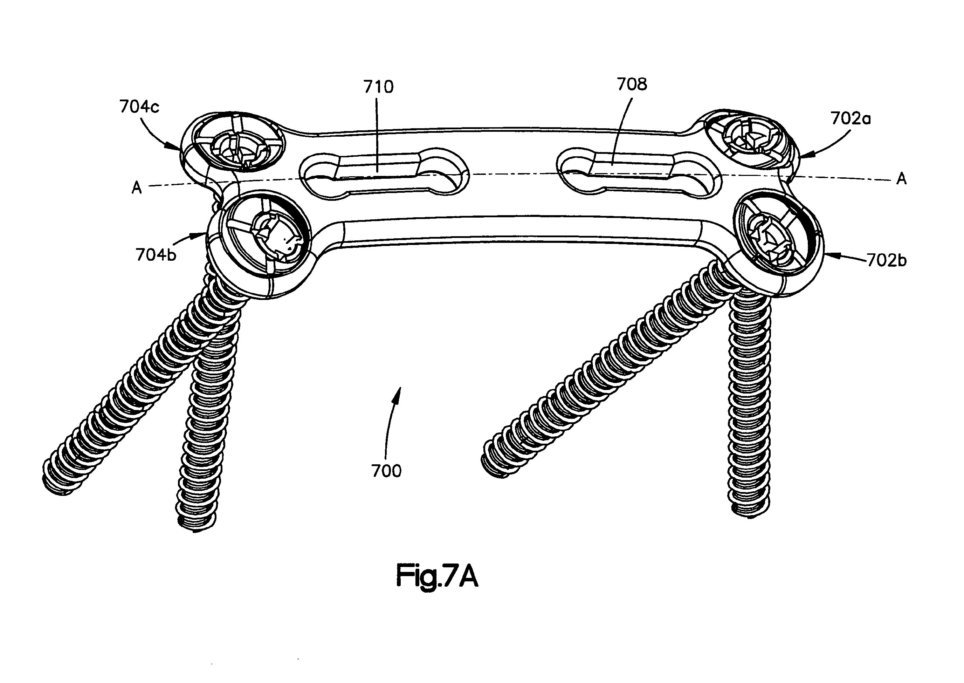

Referring to Figs. 7-9, a cervical anterior transpedicular fixation system in accordance with a second preferred embodiment of the present invention includes a plurality of rotatable eccentric compression rings 706 to accommodate insertion of bone fasteners 715.

The fixation system of the second preferred embodiment includes a plate 700 with two pairs of fixation holes 702a, 702b, 704a, 704b. Although the plate 700 is shown with two pairs of fixation holes 702a, 702b, 704a, 704b, more than two pairs may instead be provided, for example, so that the plate 700 may span a greater length and thus be fastened to multiple locations along the spine. Alternatively, single holes (not shown) may be provided in each end of the plate 700, as opposed to the pairs of fixation holes 702a, 702b, 704a, 704b.

Slots 708 and 710 are preferably defined in the plate 700 and are aligned along a central longitudinal axis A-A for receiving a drill/screw guide and for graft visualization. Preferably, the slots 708, 710 do not receive any fasteners 715, but are not so limited. Alternatively, only one slot or more than two slots may be provided, and the slot or slots may be disposed transverse to the central longitudinal axis A-A. The slots 708, 710 of the second preferred embodiment include straight portions in a central area and semicircular portions at ends of the slots 708, 710.

Each of the fixation holes 702a, 702b, 704a, 704b, which are configured to receive at least a portion of the bone fasteners 715, extends between top and bottom surfaces of the plate 700 and includes the rotatable eccentric compression ring 706. Only one or two of the fixation holes 702a, 702b, 704a, 704b may contain the rotatable eccentric compression ring 706, while the other of the holes 702a, 702b, 704a, 704b may contain no compression ring 706 at all. For example, rotatable eccentric compression rings 702a, 702b, 704a, 704b may be used in third and fourth fixation holes 702b, 704b while first and second fixation holes 702a, 704a may contain the concentric compression rings 706. Alternatively, differing shapes (not shown) of the compression rings 706, whether rotatable or fixed such as hexagonal, star-shaped etc. may be used.

Because of the eccentric nature of the compression rings 706, the screws 715 inserted into the rotatable eccentric compression rings 706 are offset from the center of the ring 706. The offset insertion and rotatable features result in both horizontal and vertical translation of the plate 700 relative to the longitudinal axis A-A. This translation is in addition to any angling of the bone screw 715 that may result from the use of the compression ring 706. For example, in addition to any translation that is enabled by the rotatable eccentricity of the compression ring 706, the compression ring 706preferably enables up to approximately twenty degrees (20°) of additional movement relative to the plate 700. Moreover, due to the rotatable eccentric compression ring 706, differences in medial-lateral entry point of the fasteners 715 can be overcome. Where entry point differences are impossible to correct, in cranial-caudal minimal and maximal distance, use of the rotatable eccentric compression rings 706 results in a small off-angle placement.

Additionally, the translation ability permitted by the rotatable eccentric nature of the compression rings 706 enables the use of a minimal amount of plates to account for a large anatomic scope. For instance, the below chart illustrates how many different size devices would be needed to cover differing anatomical distances. As will be appreciated by one of ordinary skill in the art, the minimum and maximum vertical distances covered will fluctuate based on the number of fastener holes that have the eccentric compression ring 706, the size of the plate 700 and the eccentricity of the eccentric compression ring 706, where the eccentricity of a fixation hole equals the distance between the center of the rotatable eccentric compression ring 706 in such hole and the center of the fixation hole.

**TABLE 1**

| Anatomic scope in mm between smallest and largest plate to be covered: | Number of fastener holes with eccentric features | Eccentricity in mm | Total Number of Plates Needed to cover Anatomic Scope A |
|---|---|---|---|
| ("A") | ("B") | ("C") | (A/ (2*B*C) |
| 120 | 2 | 1 | 30 |
| 110 | 2 | 1 | 28 |
| 100 | 2 | 1 | 25 |
| 90 | 2 | 1 | 23 |
| 80 | 2 | 1 | 20 |
| 70 | 2 | 1 | 18 |
| 60 | 2 | 1 | 15 |
| 50 | 2 | 1 | 13 |
| 40 | 2 | 1 | 10 |
| 30 | 2 | 1 | 8 |
| 20 | 2 | 1 | 5 |
| 10 | 2 | 1 | 3 |
| 0 | 2 | 1 | 0 |

If the eccentricity of the rotatable eccentric compression ring 706, or the number of fastener holes that have a rotatable eccentric compression ring 706 is reduced, the anatomic scope that can be covered by the associated plate 700 is also reduced. For example, as shown in Table 2 below, if the identical number of plates, as used for Table 1, were given a reduced eccentricity (by reducing the distance between the center of the rotatable eccentric compression ring 706 in one of the fixation holes and the center of such fixation hole), the plate 700 will cover a smaller area. Thus, if the same number of plates is used, a smaller anatomic scope will be covered. The below chart illustrates how many different size devices would be needed to cover the same anatomical distances as in Table 1, where the eccentricity of the rotatable eccentric compression ring 706 is reduced by twenty-five hundredths of a millimeter (0.25 mm).

**TABLE 2**

| Anatomic scope in mm between smallest and largest plate to be covered: | Number of fastener holes with eccentric features | Eccentricity in mm | Area not covered by plates in mm | Total Number of Plates Needed to cover Anatomic Scope A |
|---|---|---|---|---|
| ("A") | ("B") | ("C") | ("D") | (A / (2*B*C+D) |
| 120 | 2 | 0.75 | 0.5 | 34 |
| 110 | 2 | 0.75 | 0.5 | 31 |
| 100 | 2 | 0.75 | 0.5 | 29 |
| 90 | 2 | 0.75 | 0.5 | 26 |
| 80 | 2 | 0.75 | 0.5 | 23 |
| 70 | 2 | 0.75 | 0.5 | 20 |
| 60 | 2 | 0.75 | 0.5 | 17 |
| 50 | 2 | 0.75 | 0.5 | 14 |
| 40 | 2 | 0.75 | 0.5 | 11 |
| 30 | 2 | 0.75 | 0.5 | 9 |
| 20 | 2 | 0.75 | 0.5 | 6 |
| 10 | 2 | 0.75 | 0.5 | 3 |
| 0 | 2 | 0.75 | 0.5 | 0 |

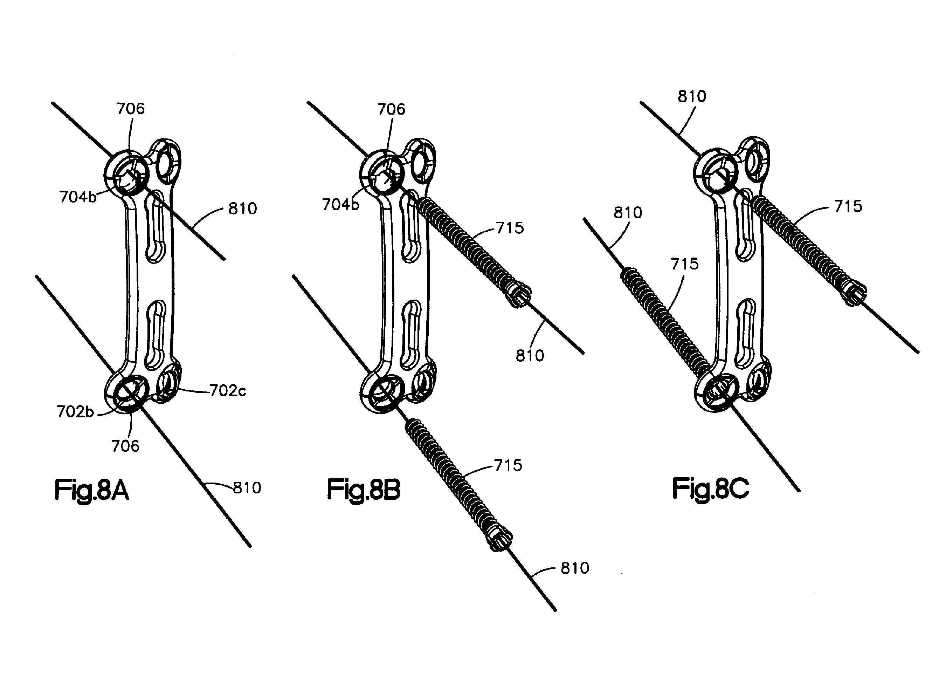

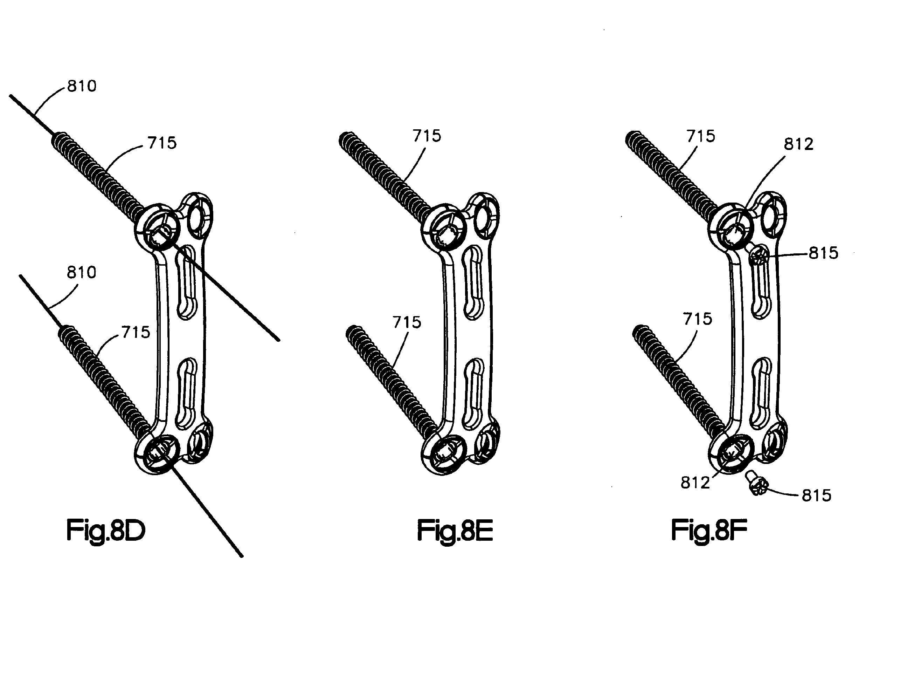

One exemplary surgical technique for implanting the plate 700 with any number of eccentric compression rings 706 is described with reference to Figs. 8A-8F. For implantation of the plate 700 in the middle and lower cervical spine, an antero-lateral approach is preferred. If the plate 700 is extended over several segments, a long incision is preferred. When exposing the vertebral bodies, the anterior longitudinal ligament is preferably removed or incised only at those points where the intervertebral disc is to be bridged by the plate 700. This selective incision preferably limits damage to the anterior adjacent segments of the anterior longitudinal ligament in adjacent segments.

After the incision has been made, an image intensifier tool, such as Fluoroscopy (not shown) may be used to guide and monitor guide wires 810, which are placed in the pedicles, preferably from the anterior side. Using spreaders, the cranial and caudal end-segments are spread and a corpectomy implant or natural bone is implanted to replace the removed segments. The distance between the guide wires 810 is measured with, for example, a caliper instrument (not shown) so that the surgeon can select a size for the plate 700.

The rotatable eccentric compression ring(s) 706 are then preferably placed around the guide wires 810 so that the plate 700 is guided to and correctly positioned against the bone. The fasteners 715, which are preferably cannulated screws, are guided over the guide wires 810 and inserted through the eccentric compression ring(s) 706. As shown in Figs. 8B-8D, only the third and fourth fastener holes 702b and 704b include eccentric compression rings 706, however, as discussed above, any number of the fastener holes may include the eccentric compression rings 706, which would vary the eccentricity of the plate 700. Although the cannulated screws 715 are preferably used, any heretofore known or hereafter developed fixation device or means of fixation can be used. For example, cortical, pedicle or spongiosa screws may be placed into the vertebral body to fasten the plate 700. In addition, the cortical or spongiosa screws may be locked using conical locking screws, as will be described in greater detail below. Once the screws 715 are inserted, the guide wires 810 are preferably removed. The head of the screw 715 may include a recess 812 for the insertion of a conical locking screw 815, which locking screw 815 can thread into the recess 812 and lock the screw 715 in place relative to the plate 700. Once the fasteners 715 have been inserted through the eccentric compression ring(s) 706 and the guide wires 810 have been removed, the locking screw 715 is preferably inserted into the head of the screw 715, thereby locking the inserted screw 715 in place.

Once the screws 715 have been inserted into the fastener holes with the rotatable eccentric compression rings 706, additional fasteners 715 are inserted into the remaining fastener holes. As with the screws 715 that are inserted into the fastener holes with rotatable eccentric compression rings 706, the screws 715 that are inserted into the remaining fastener holes may be any heretofore known or hereafter developed fixation device or means of fixation including but not limited to a screw whose head can accommodate a fixation screw as discussed above.

It should be noted that the symmetry of the plate 700 preferably allows for left and right approaches into the patient's spine. Additionally, as can be appreciated by those skilled in the art, when the plate 700 is inserted in the lumbar region of the spine, it can be combined with a plurality of anteriorly placed screws 715, which may eliminate the need for posterior instrumentation in certain circumstances. Because of the small angle between the sagittal plane and the pedicles in the human spine, the entry points of the guide wires 810 are unlikely to cross each other on the anterior vertebra.

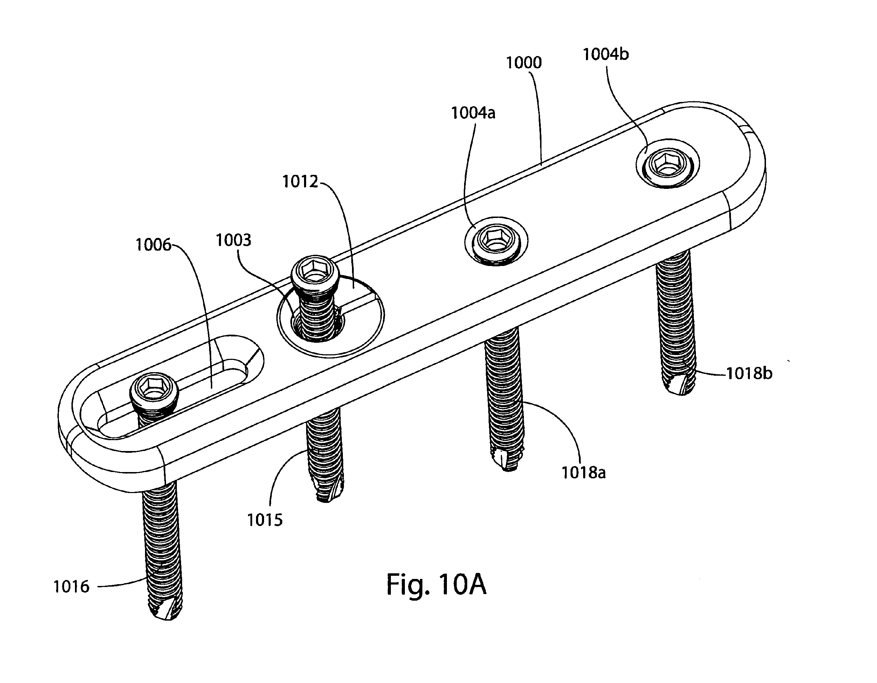

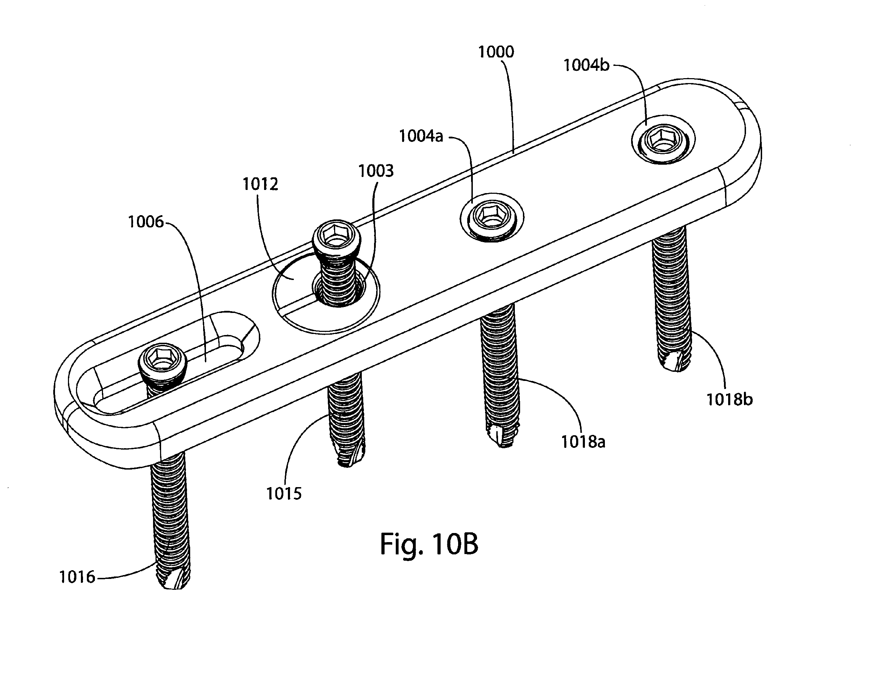

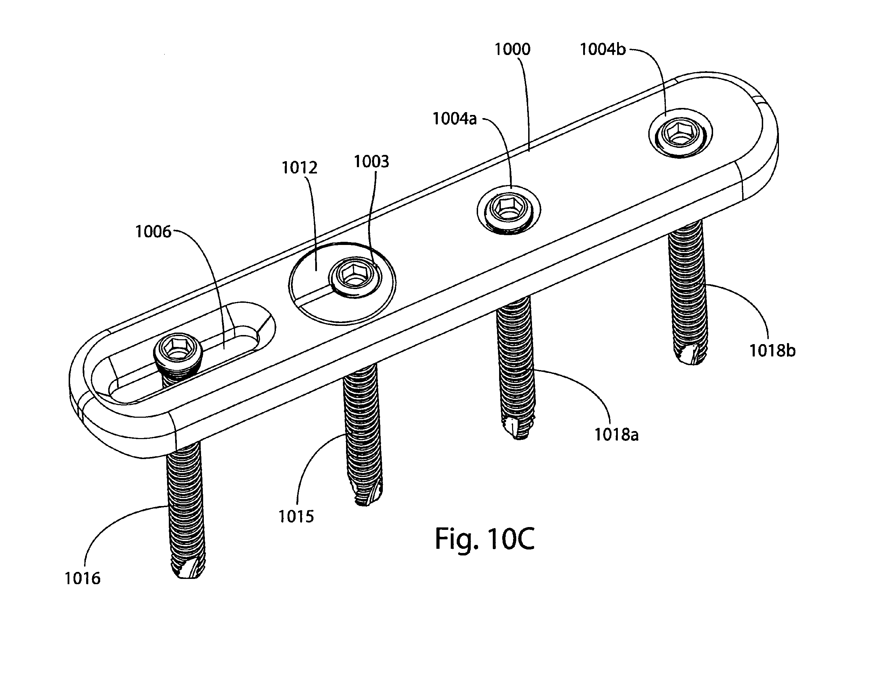

Referring to Figs. 10A-10C, a third preferred embodiment of a bone plate fixation system includes a rotatable eccentric compression ring 1012 and a slot-shaped fixation hole 1006 for insertion of the bone fasteners 1015, 1016, which enables additional compression of a fracture gap after the fasteners 1015, 1016, 1018a, 1018b have been placed in a bone plate 1000.

The fixation system of the third preferred embodiment includes the bone plate 1000 with first and second fixation holes 1003 1004a, 1004b and a slot-shaped fixation hole 1006. Although the plate 1000 is shown with four fixation holes 1003 1004a, 1004b, 1006, more or less than the four fixation holes 1003 1004a, 1004b, 1006 may be included in the plate 1000, for example, so that the plate 1000 may span a greater or lesser length and thus be fastened to different and/or multiple locations along a bone fragment. The bone plate 1000 preferably includes at least two fasteners 1015, 1016, 1018a, 1018b for each bone fragment.

The slot-shaped fixation hole 1006 preferably allows translation of the fastener 1016, in situ. Each of the fixation holes 1006, 1003 1004a, 1004b, which may be configured to receive at least a portion of one of the bone fasteners 1016, 1015, 1018a, 1018b, respectively, extends between the top and bottom surfaces of the plate 1000 in a mounted position. The first fixation hole 1003 preferably includes the rotatable eccentric compression ring 1012. Multiple of the fixation holes 1006, 1003 1004a, 1004b may contain the rotatable eccentric compression ring 1012, while other of the fixation holes 1006, 1003 1004a, 1004b may also contain the concentric compression ring 1012 or no compression ring at all. Alternatively, differing shapes of the compression rings 1012, whether rotatable or fixed, such as hexagonal, star-shaped etc. may be used.

Because of the eccentric nature of the compression ring 1012, the screw 1015 is offset from the center of such the compression ring 1012. The offset insertion and rotatable features result in horizontal and/or vertical translation of the plate 1000 relative to its longitudinal axis. This translation is in addition to any angling of the bone screw 1015 that may result from the use of the compression ring 1012. For example, in addition to any translation that is enabled by the rotatable eccentricity of the compression ring 1012, the compression ring 1012 preferably enables up to approximately twenty degrees (20°) of additional movement relative to the plate 1000. Additionally, as can be seen in Figs 10A-10C, the fastener 1015 may have a conical threaded head, which when inserted into the fixation hole 1003, can expand the compression ring 1012, thereby locking the fastener 1015 in place.

When the plate 1000 is to be implanted over a fracture site to span a gap between bone fragments, the fastener holes 1004a, 1004b are placed on one side of the gap and the fastener holes 1006, 1003 are placed on the other side of the gap or the affected disc space and fixed to the bone using fasteners 1016, 1018a, 1018b, Once the fastener 105 has been partially inserted into the fixation hole 1003, at the surgeon's option, the compression ring 1012 can be rotated up to one hundred eighty degrees (180°) which allows the surgeon to compress the fracture gap, as a corrective action, without affecting the alignment of the fragments. Once the proper position for the fastener 1015 has been selected and the compression ring 1012 is appropriately rotated, the fastener 1015 is then implanted.

Those skilled in the art will recognize that the method and system of the present invention has many applications, may be implemented in many manners and, as such is not to be limited by the foregoing embodiments and examples. Any number of the features of the different embodiments described herein may be combined into one single embodiment and alternate embodiments having fewer than or more than all of the features herein described are possible. Functionality may also be, in whole or in part, distributed among multiple components, in manners now known or to become known. Moreover, the scope of the present invention covers conventionally known and features of those variations and modifications through the components described herein as would be understood by those skilled in the art. It is the intention, therefore, to be limited only as indicated by the scope of the claims appended hereto.

It is also to be understood that the following claims are intended to cover all of the generic and specific features of the invention herein described and all statements of the scope of the invention, which, as a matter of language, might be said to fall therebetween.

It will be appreciated by those skilled in the art that changes could be made to the embodiments described above without departing from the broad inventive concept thereof. It is understood, therefore, that this invention is not limited to the particular embodiments disclosed, but it is intended to cover modifications within the spirit and scope of the present invention as defined by the appended claims.

## Claims

1. A bone fixation system comprising:
a plate (700;1000) having a central longitudinal axis, the plate (700;1000) including a top surface, a bottom surface and at least one fixation hole (702a; 702b; 704a; 704b; 1003; 1004a; 1004b; 1006) extending between the top and bottom surfaces;
at least one rotatable eccentric compression ring (706;1012) disposed in at least a portion of the at least one fixation hole (702a;702b;704a;704b;1003; 1004a; 1004b; 1006); and
at least one fastener (715;1015;1016;1018a;1018b) including a head and a threaded shaft, the at least one rotatable eccentric compression ring (706;1012) is configured and dimensioned to permit the plate (700;1000) to translate relative to the at least one fastener(715;1015;1016;1018a;1018b).

2. The bone fixation system of claim 1 further comprising a slot (708;710) extending through the central longitudinal axis of the plate (700).

3. The bone fixation system of claim 1 or 2, wherein the plate (700;1000) has a plurality of fixation holes (702a;702b;704a;704b;1003;1004a;1004b; 1006) and at least one of said plurality of fixation holes has a concentric compression ring disposed therein.

4. The bone fixation system of any of claims 1 to 3, wherein the at least one fastener (715) comprises: a threaded shaft and a head, the head including a radial wall and an open end defining a recess (812) for insertion of a threaded locking screw (815) wherein when the threaded locking screw (815) is threaded into the head of the fastener (715), the radial wall is expanded outward to interact with a wall of the eccentric compression ring.

5. The bone fixation system of any of claims 1 to 4, wherein the rotatable eccentric compression ring (706;1012) enables up to 20° additional movement relative to the plate (700;1000).

6. The bone fixation system of any of claims 1 to 5, wherein the bone plate fixation system includes a slot-shaped fixation hole (1006) for insertion of a bone fastener (1016) so as to allow translation of the fastener (1016) in situ.

7. The bone fixation system to any of claims 1 to 6, wherein the fastener (1015) has a conical threaded head, which when inserted into the fixation hole (1003) can expand the compression ring (1012).
